(19) **Europäisches Patentamt / European Patent Office / Office européen des brevets**

(11) **EP 4 310 497 A1**

(12) **EUROPEAN PATENT APPLICATION**
published in accordance with Art. 153(4) EPC

(43) Date of publication:
**24.01.2024 Bulletin 2024/04**

(21) Application number: **21931736.9**

(22) Date of filing: **17.12.2021**

(51) International Patent Classification (IPC):
**G01N 33/543** (2006.01)   **G01N 21/41** (2006.01)
**G01N 21/55** (2014.01)

(52) Cooperative Patent Classification (CPC):
**G01N 15/00; G01N 21/41; G01N 21/55;
G01N 33/543;** G01N 2015/0038

(86) International application number:
**PCT/JP2021/046699**

(87) International publication number:
**WO 2022/195994 (22.09.2022 Gazette 2022/38)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **18.03.2021   JP 2021044920
18.03.2021   JP 2021044933**

(71) Applicant: **JVCKenwood Corporation
Yokohama-shi, Kanagawa 2210022 (JP)**

(72) Inventors:
• **SAITO Atsushi**
  **Yokohama-shi, Kanagawa 221-0022 (JP)**
• **ITONAGA Makoto**
  **Yokohama-shi, Kanagawa 221-0022 (JP)**
• **ONO Masayuki**
  **Yokohama-shi, Kanagawa 221-0022 (JP)**

(74) Representative: **Schmidbauer, Andreas Konrad
Wagner & Geyer Partnerschaft mbB
Patent- und Rechtsanwälte
Gewürzmühlstrasse 5
80538 München (DE)**

(54) **ANALYSIS DEVICE AND ANALYSIS METHOD**

(57)   A controller (9) divides a reaction region into a plurality of unit sections. The controller (9) generates first array data of a measured aggregate value of unit sections in which no nanoparticles exist, a measured aggregation value of unit sections in which a single nanoparticle exists, and measured aggregation values of unit sections in which 2 to n close nanoparticles exist. The controller (9) generates second array data of a theoretical aggregate value of unit sections in which no nanoparticles exist, a theoretical aggregation value of unit sections in which a single nanoparticle exists, and theoretical aggregation values of unit sections in which 2 to n close nanoparticles exist, based on a probability distribution according to a probability theory, in which the second array data most closely approximates the first array data. The controller (9) generates a count value of the nanoparticles in the reaction region, based on the theoretical aggregation value of unit sections in which the single nanoparticle exists and the theoretical aggregation values of unit sections in which the 2 to n close nanoparticles exist.

FIG. 9

**Description**

[TECHNICAL FIELD]

The present disclosure relates to an analysis device and analysis method for analyzing biological substances such as antigens or antibodies.

[BACKGROUND ART]

Patent Literature 1 discloses an analysis device for counting biological substances fixed to an optical disc as detection target substances. A plurality of antibodies are fixed to the optical disc. The detection target substances are fixed to the optical disc by binding antigens, which are the detection target substances, to the antibodies. Nanoparticles bind to the respective antigens as labels. The nanoparticles are called nanobeads and are larger than the antigens. The analysis device indirectly counts the detection target substances by irradiating the optical disc with laser light and detecting the number of nanobeads based on the reflected light from the optical disc.

It is not always the case that a plurality of detection target substances labeled with nanobeads are fixed in a state in which individual detection target substances are sufficiently separated from other detection target substances on an optical disc. Two or more nanobeads may be fixed on the optical disc in close proximity to each other (including in contact with each other) at a very small distance. When two or more nanobeads are close to each other, light-reception level signals indicating the light-reception level of the reflected light optically interfere with each other. The analysis device disclosed in Patent Literature 1 is configured to correctly count nanobeads even when two or more nanobeads are close to each other and the light-reception level signals interfere with each other.

[CITATION LIST]

[PATENT LITERATURE]

Patent Literature 1: Japanese Unexamined Patent Application Publication No. 2020-20668

[SUMMARY OF INVENTION]

When the surface of an optical disc is divided into unit sections of a predetermined area, the number of detection target substances, to which nanobeads are bound, fixed in each unit section (that is, the number of nanobeads) theoretically follows a probability distribution according to a probability theory. Therefore, originally, the number of nanobeads (0, 1, 2 ...) which are distributed in each unit section depends on the concentration of the detection target substances in a sample solution when the detection target substances are fixed to the optical disc by injecting the sample solution containing the detection target substances into wells arranged on the optical disc.

However, in practice, the number of nanobeads which are distributed in each unit section does not exactly depend on the concentration of the detection target substances in the sample solution, contrary to the probability theory. This is because there may be two or more nanobeads in an aggregated state in the unit section because of the aggregation-prone nature of nanobeads.

Further, noise (hereinafter referred to as assay noise) generated during the process (assay) of fixing a specimen or beads on the optical disc may be incorrectly counted as nanobeads. For example, assay noise may result from the precipitation of salts or other components in a cleaning solution or reagent, or from the removal of beads when a pipette tip for injecting a reagent touches the optical disc during the assay process. In addition, assay noise may result when various noise components (such as protein clusters) contained in the reagent are attached to the optical disc and detected as signals. Such assay noise may be erroneously counted as nanobeads.

Accordingly, when nanobeads (detection target substances) are counted by the analysis device described in Patent Literature 1, the detection target substances may not be counted correctly due to erroneously counting aggregated nanobeads or assay noise.

An object of one or more embodiments is to provide an analysis device and an analysis method capable of, compared to the past, more correctly counting single nanoparticles which exist alone and are not close to other nanoparticles within an interference distance within which there is optical interference with other nanoparticles, and two or more close nanoparticles which are close to each other within the interference distance, and further avoiding the influence of aggregation of the nanoparticles, which label a detection target substance, and assay noise.

If it is possible to quantitatively evaluate the degree of aggregation of nanoparticles which label a detection target substance, it becomes possible to improve the quality of the nanoparticles and contribute to the development of a method for suppressing the aggregation. For this reason, it is desirable to develop an analysis device and an analysis method which can quantitatively evaluate the degree of aggregation of nanoparticles. Therefore, an object of one or more embodiments is to further provide an analysis device and an analysis method capable of quantitatively evaluating the degree of aggregation of nanoparticles which label a detection target substance.

A first aspect of one or more embodiments provides an analysis device including: an optical pickup configured to irradiate a sample analysis disc with a laser beam and to detect a light-reception level of reflected light from a reaction region to generate a light-reception level signal, the sample analysis disc having the reaction region, and the reaction region having fixed thereto a plurality of detection target substances having nanoparticles that are labels bound thereto; a pulse detection circuit configured to detect, from a waveform of the light-reception level signal, a single pulse waveform indicating a single nanoparticle that exists alone, and $(n - 1)$ types of close pulse waveforms indicating 2 to n close nanoparticles (n being an integer) in which adjacent nanoparticles are close to each other within an interference distance, thereby generating respective detection values; and a nanoparticle counter configured to generate a count value of the nanoparticles in the reaction region.

The nanoparticle counter divides the reaction region into a plurality of unit sections; based on the respective detection values, individually aggregates the number of unit sections in which the single pulse waveform is detected, and the number of unit sections in which the close pulse waveforms are detected with respect to each of the $(n - 1)$ types, and generates a measured aggregation value of unit sections in which the single nanoparticle exists alone and respective measured aggregation values of unit sections in which the 2 to n close nanoparticles exist; and calculates the number of unit sections in which neither of the single pulse waveform and the close pulse waveforms are detected by subtracting, from the number of all unit sections in the reaction region, the number of unit sections in which the single pulse waveform is detected and the number of unit sections in which the close pulse waveforms are detected with respect to each of the $(n - 1)$ types, thereby generating a measured aggregate value of unit sections in which no nanoparticles exist.

Further, the nanoparticle counter generates first array data of the measured aggregate value of unit sections in which no nanoparticles exist, the measured aggregation value of unit sections in which the single nanoparticle exists, and the measured aggregation values of unit sections in which the 2 to n close nanoparticles exist; generates or selects second array data of a theoretical aggregate value of unit sections in which no nanoparticles exist, a theoretical aggregation value of unit sections in which the single nanoparticle exists, and theoretical aggregation values of unit sections in which the 2 to n close nanoparticles exist, the second array data being array data of theoretical aggregate values based on a probability distribution according to a probability theory and most closely approximating the first array data; and calculates the number of nanoparticles in the reaction region, based on the theoretical aggregation value of unit sections in which the single nanoparticle exists and the theoretical aggregation values of unit sections in which the 2 to n close nanoparticles exist.

A second aspect of one or more embodiments provides an analysis method including: irradiating a sample analysis disc with a laser beam by means of an optical pickup, the sample analysis disc having a reaction region to which there are fixed a plurality of detection target substances having nanoparticles that are labels bound thereto; detecting a light-reception level of reflected light from the reaction region and generating a light-reception level signal by means of the optical pickup; and detecting, from a waveform of the light-reception level signal, a single pulse waveform indicating a single nanoparticle that exists alone, and $(n - 1)$ types of close pulse waveforms indicating 2 to n close nanoparticles (n being an integer) in which adjacent nanoparticles are close to each other within an interference distance, thereby generating respective detection values, by means of a pulse detection circuit; in which the analysis method includes, by means of a controller that obtains the respective detection values, dividing the reaction region into a plurality of unit sections; based on the respective detection values, individually aggregating the number of unit sections in which the single pulse waveform is detected, and the number of unit sections in which the close pulse waveforms are detected with respect to each of the $(n - 1)$ types, and generating a measured aggregation value of unit sections in which the single nanoparticle exists alone and respective measured aggregation values of unit sections in which the 2 to n close nanoparticles exist; calculating the number of unit sections in which neither of the single pulse waveform and the close pulse waveforms are detected by subtracting, from the number of all unit sections in the reaction region, the number of unit sections in which the single pulse waveform is detected and the number of unit sections in which the close pulse waveforms are detected with respect to each of the $(n - 1)$ types, thereby generating a measured aggregate value of unit sections in which no nanoparticles exist; generating first array data of the measured aggregate value of unit sections in which no nanoparticles exist, the measured aggregation value of unit sections in which the single nanoparticle exists, and the measured aggregation values of unit sections in which the 2 to n close nanoparticles exist; generating or selecting second array data of a theoretical aggregate value of unit sections in which no nanoparticles exist, a theoretical aggregation value of unit sections in which the single nanoparticle exists, and theoretical aggregation values of unit sections in which the 2 to n close nanoparticles exist, the second array data being array data of theoretical aggregate values based on a probability distribution according to a probability theory and most closely approximating the first array data; and calculating the number of nanoparticles in the reaction region, based on the theoretical aggregation value of unit sections in which the single nanoparticle exists and the theoretical aggregation values of unit sections in which the 2 to n close nanoparticles exist, thereby generating a count value of the nanoparticles in the reaction region.

A third aspect of one or more embodiments provides an analysis device including: an optical pickup configured to irradiate a sample analysis disc with a laser beam and to detect a light-reception level of reflected light from a reaction region to generate a light-reception level signal, the sample analysis disc having the reaction region, and the reaction region having fixed thereto a plurality of detection target substances having nanoparticles that are labels bound thereto; a pulse detection circuit configured to detect, from a waveform of the light-reception level signal, a single pulse waveform indicating a single nanoparticle that exists alone, and (n - 1) types of close pulse waveforms indicating 2 to n close nanoparticles (n being an integer) in which adjacent nanoparticles are close to each other within an interference distance, thereby generating respective detection values; and an aggregation degree generating unit configured to calculate an aggregation degree indicating a degree of aggregation of the nanoparticles in the reaction region.

The aggregation degree generating unit divides the reaction region into a plurality of unit sections; based on the respective detection values, individually aggregates the number of unit sections in which the single pulse waveform is detected, and the number of unit sections in which the close pulse waveforms are detected with respect to each of the (n - 1) types, and generates a measured aggregation value of unit sections in which the single nanoparticle exists alone and respective measured aggregation values of unit sections in which the 2 to n close nanoparticles exist; and calculates the number of unit sections in which neither of the single pulse waveform and the close pulse waveforms are detected by subtracting, from the number of all unit sections in the reaction region, the number of unit sections in which the single pulse waveform is detected and the number of unit sections in which the close pulse waveforms are detected with respect to each of the (n - 1) types, thereby generating a measured aggregate value of unit sections in which no nanoparticles exist.

Further, the aggregation degree generating unit generates first array data of the measured aggregate value of unit sections in which no nanoparticles exist, the measured aggregation value of unit sections in which the single nanoparticle exists, and the measured aggregation values of unit sections in which the 2 to n close nanoparticles exist; generates or selects second array data of a theoretical aggregate value of unit sections in which no nanoparticles exist, a theoretical aggregation value of unit sections in which the single nanoparticle exists, and theoretical aggregation values of unit sections in which the 2 to n close nanoparticles exist, the second array data being array data of theoretical aggregate values based on a probability distribution according to a probability theory and most closely approximating the first array data; and divides a quantitative difference between the first array data and the second array data by a count value obtained by calculating the number of nanoparticles in the reaction region, based on the measured aggregation value of unit sections in which the single nanoparticle exists and the measured aggregation values of unit sections in which the 2 to n close nanoparticles exist.

A fourth aspect of one or more embodiments provides an analysis method including: irradiating a sample analysis disc with a laser beam by means of an optical pickup, the sample analysis disc having a reaction region to which there are fixed a plurality of detection target substances having nanoparticles that are labels bound thereto; detecting a light-reception level of reflected light from the reaction region and generating a light-reception level signal by means of the optical pickup; and detecting, from a waveform of the light-reception level signal, a single pulse waveform indicating a single nanoparticle that exists alone, and (n - 1) types of close pulse waveforms indicating 2 to n close nanoparticles (n being an integer) in which adjacent nanoparticles are close to each other within an interference distance, thereby generating respective detection values, by means of a pulse detection circuit; in which the analysis method includes, by means of a controller that obtains the respective detection values, dividing the reaction region into a plurality of unit sections; based on the respective detection values, individually aggregating the number of unit sections in which the single pulse waveform is detected, and the number of unit sections in which the close pulse waveforms are detected with respect to each of the (n - 1) types, and generating a measured aggregation value of unit sections in which the single nanoparticle exists alone and respective measured aggregation values of unit sections in which the 2 to n close nanoparticles exist; calculating the number of unit sections in which neither of the single pulse waveform and the close pulse waveforms are detected by subtracting, from the number of all unit sections in the reaction region, the number of unit sections in which the single pulse waveform is detected and the number of unit sections in which the close pulse waveforms are detected with respect to each of the (n - 1) types, thereby generating a measured aggregate value of unit sections in which no nanoparticles exist; generating first array data of the measured aggregate value of unit sections in which no nanoparticles exist, the measured aggregation value of unit sections in which the single nanoparticle exists, and the measured aggregation values of unit sections in which the 2 to n close nanoparticles exist; generating or selecting second array data of a theoretical aggregate value of unit sections in which no nanoparticles exist, a theoretical aggregation value of unit sections in which the single nanoparticle exists, and theoretical aggregation values of unit sections in which the 2 to n close nanoparticles exist, the second array data being array data of theoretical aggregate values based on a probability distribution according to a probability theory and most closely approximating the first array data; and dividing a quantitative difference between the first array data and the second array data by a count value obtained by calculating the number of nanoparticles in the reaction region, based on the measured aggregation value of unit sections in which the single nanoparticle exists and the measured aggregation values of unit sections in which the 2 to n close nanoparticles exist, thereby calculating an aggregation degree indicating a degree of aggregation of the nanoparticles in the reaction region.

The analysis device and the analysis method according to one or more embodiments make it possible to, compared to the past, more correctly count single nanoparticles which exist alone and are not close to other nanoparticles within an interference distance within which there is optical interference with other nanoparticles, and two or more close nanoparticles which are close to each other within the interference distance, and to further avoid the influence of aggregation of the nanoparticles, which label a detection target substance, and assay noise. In addition, the analysis device and the analysis method according to one or more embodiments make it possible to quantitatively evaluate the degree of aggregation of nanoparticles which label a detection target substance.

[BRIEF DESCRIPTION OF DRAWINGS]

FIG. 1 is a plan view of a detection target substance capture unit as viewed from a cartridge side.

FIG. 2 is a plan view of the detection target substance capture unit as viewed from a sample analysis disc side.

FIG. 3 is a cross-sectional view of the detection target substance capture unit illustrated in FIG. 1 cut along a line A-A.

FIG. 4 is a cross-sectional view illustrating a state in which the cartridge is removed from the sample analysis disc.

FIG. 5 is an enlarged perspective view partially illustrating a spiral-shaped recess and a spiral-shaped protrusion formed on a surface of the sample analysis disc in a state in which the sample analysis disc is broken.

FIG. 6 is a plan view conceptually illustrating the surface of the sample analysis disc in a well provided in the detection target substance capture unit.

FIG. 7A is a partially enlarged cross-sectional view illustrating a state in which antibodies are bound to the protrusions and recesses on a bottom surface of the well.

FIG. 7B is a partially enlarged cross-sectional view illustrating a state in which a detection target substance is bound to an antibody.

FIG. 7C is a partially enlarged cross-sectional view illustrating a state in which a nanoparticle is bound to the detection target substance.

FIG. 8 is a plan view conceptually illustrating a state in which a plurality of nanoparticles are caught in the recesses in the well.

FIG. 9 is a configuration diagram illustrating an analysis device according to first to third embodiments.

FIG. 10 is a waveform diagram illustrating an example of waveforms of a light-reception level signal for a single nanoparticle, a double nanoparticle, and a triple nanoparticle.

FIG. 11 is a partially enlarged plan view illustrating a state in which a reaction region is divided into a plurality of mesh-like unit sections.

FIG. 12 is a block diagram illustrating a controller of the analysis device according to the first embodiment having a nanoparticle counter as a functional configuration.

FIG. 13 is a flowchart illustrating processing executed by the analysis device according to the first embodiment, and an analysis method according to the first embodiment.

FIG. 14 is a diagram illustrating array data of measured aggregate values and theoretical aggregate values for no nanoparticles, a single nanoparticle, a double nanoparticle, and a triple nanoparticle.

FIG. 15 is a diagram illustrating a relationship between a sample concentration of the detection target substance, and count values of the nanoparticles based on measured aggregate values obtained at each sample concentration and count values of the nanoparticles based on theoretical aggregate values obtained at each sample concentration.

FIG. 16 is a block diagram illustrating a controller of the analysis device according to the second embodiment having an aggregation generating unit as a functional configuration.

FIG. 17 is a flowchart illustrating processing executed by the analysis device according to the second embodiment, and an analysis method according to the second embodiment.

FIG. 18 is a diagram illustrating an example in which a relationship between count values and aggregation degrees of the nanoparticles is plotted.

[DESCRIPTION OF EMBODIMENTS]

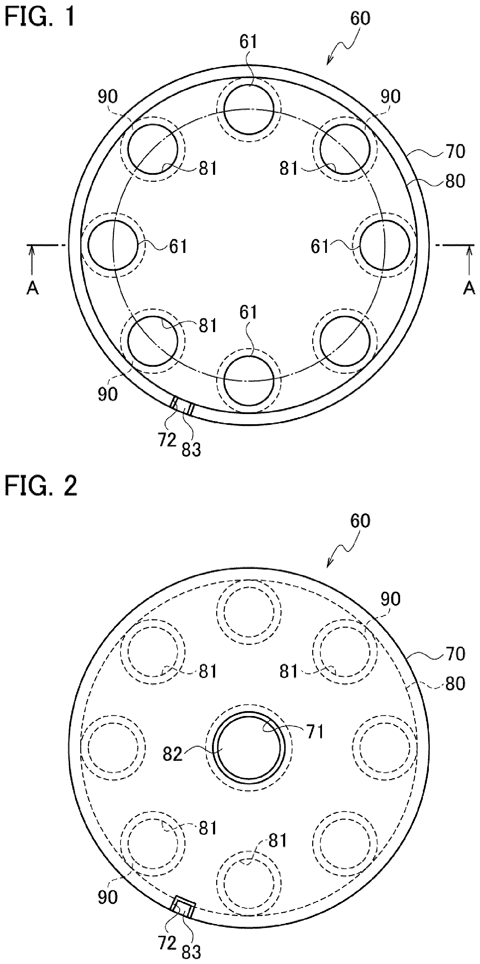

Hereinafter, an analysis device and an analysis method according to first and second embodiments will be described with reference to the accompanying drawings. Before describing a configuration and an operation of the analysis device according to the first and second embodiments, a description will be given with reference to FIGS. 1 to 6 regarding how to prepare a sample analysis disc, which is an optical disc to be analyzed by the analysis device according to the first and second embodiments and in which detection target substances are fixed onto the surface of the sample analysis disc.

[Configuration of detection target substance capture unit]

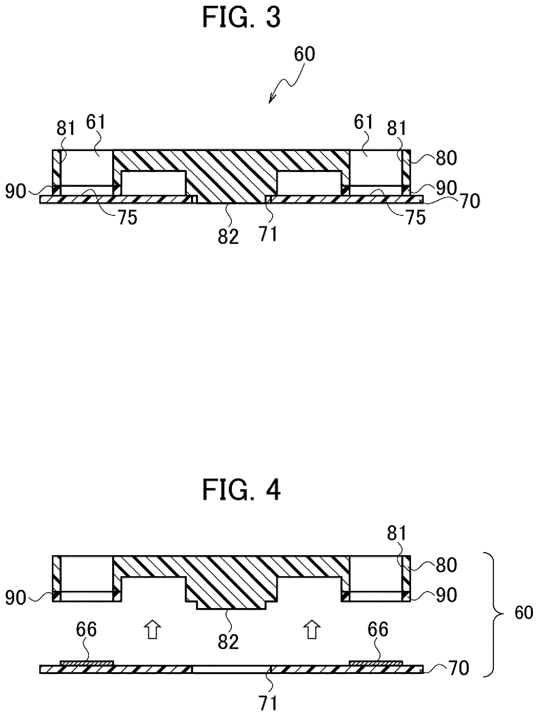

FIG. 1 illustrates a state in which a detection target substance capture unit 60 (hereinafter, a capture unit 60) is viewed from a cartridge 80 side. The substance capture unit 60 is for preparing a sample analysis disc 70 in which the detection target substances are fixed onto the surface thereof. FIG. 2 illustrates a state in which the capture unit 60 is viewed from the sample analysis disc 70 side. FIG. 3 illustrates a cross section of the capture unit 60 cut along a line A-A of FIG. 1. FIG. 4 illustrates a state in which the cartridge 80 is removed from the sample analysis disc 70.

As illustrated in FIGS. 1 to 4, the capture unit 60 includes the sample analysis disc 70, the cartridge 80, and a seal member 90. The sample analysis disc 70 has a disc shape equivalent to an optical disc such as, a Blu-ray Disc (BD), a DVD, or a compact disc (CD). The sample analysis disc 70 is formed of a resin material such as a polycarbonate resin or a cycloolefin polymer commonly used in optical discs. Note that the sample analysis disc 70 is not limited to the optical disc described above, and may be in another form. For example, an optical disc conforming to another prescribed standard may be used.

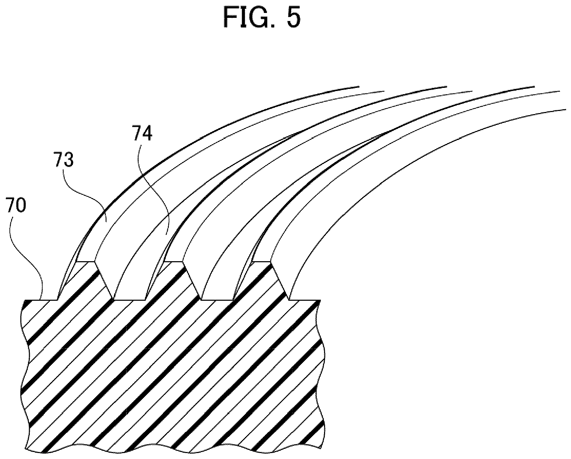

FIG. 5 is a partially enlarged view of the surface of the sample analysis disc 70. As illustrated in FIG. 5, on the surface of the sample analysis disc 70, one protrusion 73 and one recess 74 are formed adjacent to each other in a spiral shape from the inner periphery to the outer periphery. The protrusion 73 corresponds to a land of an optical disc, and the recess 74 corresponds to a groove of an optical disc.

One circumference at each position in the radial direction of the spiral-shaped protrusion 73 and the spiral-shaped recess 74 adjacent to each other is one track. That is, one track is when scanning of the recess 74 performed by irradiation of a laser beam starts at a position in the circumferential direction, the sample analysis disc 70 rotates by 360 degrees, and the laser beam reaches the same position in the circumferential direction although the position in the radial direction is deviated. A track pitch corresponding to the pitch of the recess 74 in the radial direction is 320 nm, for example.

As illustrated in FIGS. 2 to 4, the sample analysis disc 70 has a center hole 71 formed in the central portion and a notch 72 formed in the outer peripheral portion. The notch 72 is a reference position recognition unit for recognizing a reference position of the sample analysis disc 70. The reference position recognition unit may be formed by something other than the notch 72.

As illustrated in FIG. 1, the cartridge 80 has a plurality of cylindrical through-holes 81 formed in the circumferential direction. The plurality of through-holes 81 are formed at equal intervals such that the respective centers are positioned on the same circumference. As illustrated in FIGS. 1 to 4, the cartridge 80 has a protrusion 82 formed at the center thereof and a protrusion 83 formed at the outer periphery thereof. By inserting the protrusion 82 into the center hole 71 of the sample analysis disc 70 and inserting the protrusion 83 into the notch 72, the cartridge 80 is positioned on the sample analysis disc 70, and thus the cartridge 80 and the sample analysis disc 70 are integrated with each other.

As illustrated in FIG. 3, the seal member 90 is arranged between the cartridge 80 and the sample analysis disc 70. The seal member 90 is ring-shaped packing made of an elastically deformed member such as silicone rubber. The seal member 90 is arranged around each through-hole 81. When the cartridge 80 is attached to the sample analysis disc 70, the seal member 90 elastically deforms to fill the recess 74 formed on the surface of the sample analysis disc 70.

As illustrated in FIGS. 1 and 3, in a state in which the cartridge 80 is attached to the sample analysis disc 70, the capture unit 60 has a plurality of cylindrical wells 61 that are formed of the through-holes 81, the seal members 90, and the surface of the sample analysis disc 70. The inner peripheral surface of the through-hole 81 and the seal member 90 forms the inner peripheral surface of the well 61, and the surface of the sample analysis disc 70 forms the bottom surface of the well 61. The well 61 functions as a container for storing a solution such as a sample solution or a buffer solution. Since the cartridge 80 adheres to the surface of the sample analysis disc 70 by means of the sealing member 90, there is hardly no leakage of the solution from the well 61.

Although the capture unit 60 illustrated in FIG. 1 includes eight wells 61, at least one well 61 is required, and the number of wells 61 is not particularly limited.

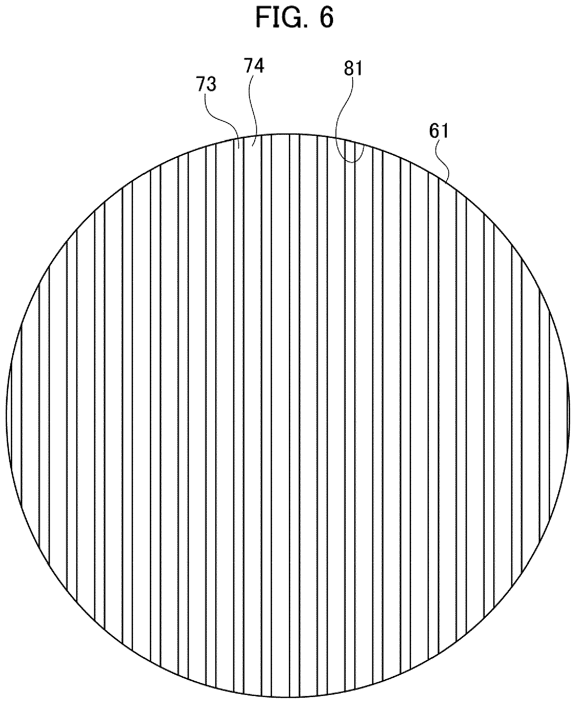

FIG. 6 conceptually illustrates the surface of the sample analysis disc 70 in the well 61. On the bottom surface of each well 61, there are a plurality of partial tracks in the circumferential direction, which constitute some tracks from among all of the tracks formed on the sample analysis disc 70. Strictly speaking, the plurality of tracks on the bottom surface of each well 61 are approximately arc-shaped. FIG. 6 is an enlarged view of the protrusion 73 and the recess 74, and there are about 20,000 tracks in each well 61.

[Forming method of reaction region]

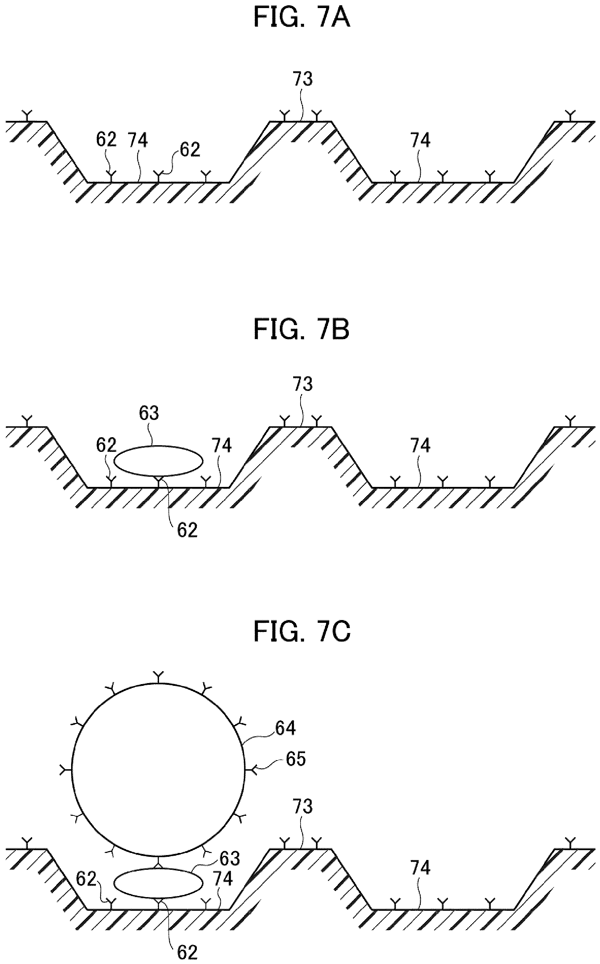

Using the capture unit 60 configured as described above, the operator injects a buffer solution containing antibodies 62 (see FIG. 7A) into the well 61 and incubates the buffer solution. Accordingly, as illustrated in FIG. 7A, the antibodies 62 are fixed to the protrusions 73 and the recesses 74 on the bottom surface of the well 61. The operator discharges the buffer solution, washes the inner side of the well 61, and injects a sample solution containing the detection target substance 63 which is an antigen (see FIG. 7B), into the well 61 and incubates the sample solution. Accordingly, as illustrated in FIG. 7B, the detection target substance 63 binds specifically to an antibody 62 due to an antigen-antibody reaction with the antibody 62.

The detection target substance 63 is a discretionary biological substance such as an antigen or an antibody, and is typically an exosome. Exosomes are present in body fluids such as blood. If blood is used as a sample, the sample solution is a liquid containing blood. Exosomes have a size of about 100 nm.

The operator discharges the sample solution, washes the inner side of the well 61, and injects a buffer solution containing the nanoparticle 64 which is a label (see FIG. 7C) into the well 61 and incubates the buffer solution. The nanoparticle 64 may be referred to as a nanobead. Antibodies 65 that bind specifically due to an antigen-antibody reaction with the detection target substance 63 are fixed onto the surface of nanoparticle 64. Accordingly, as illustrated in FIG. 7C, the nanoparticle 64 is caught in the recess 74 in a state in which the nanoparticle 64 binds to the detection target substance 63. The nanoparticle 64 has a size of about 200 nm.

In FIG. 7B, the detection target substance 63 may bind to the antibodies 62 fixed to the protrusion 73; however, in most cases, the detection target substance 63 on the protrusion 73 is removed by washing the inner side of the well 61. Thus, in FIG. 7C, most of the nanoparticles 64 are caught in the recess 74.

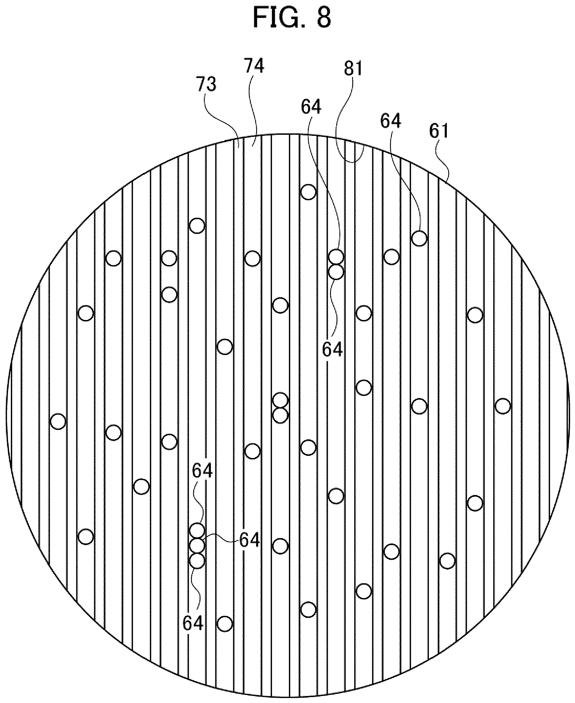

FIG. 8 illustrates a state in which a plurality of nanoparticles 64 are caught in the recess 74 in the well 61. Although the plurality of nanoparticles 64 are mainly dispersed in the plurality of tracks and fixed thereto, two or more nanoparticles 64 are fixed in the recess 74 in close proximity to each other at a very short distance or in contact with each other at a very short distance.

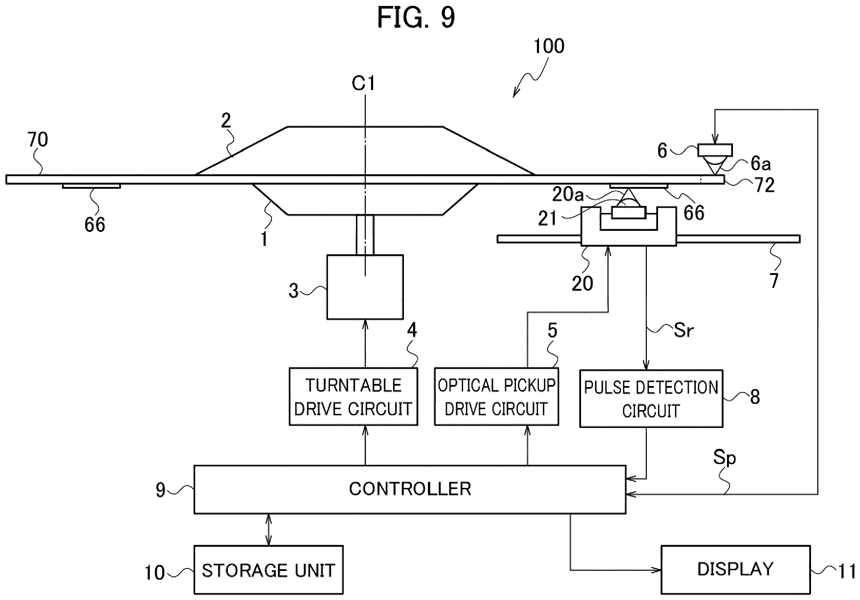

The operator discharges a buffer solution containing the nanoparticles 64 and washes the inner side of the well 61. Thereafter, as illustrated in FIG. 4, the operator removes the cartridge 80 and the sealing member 90 from the sample analysis disc 70. A reaction region 66 is formed on the surface of the sample analysis disc 70, corresponding to the position of the well 61. In the reaction region 66, a plurality of nanoparticles 64 that bind to the detection target substance 63 are caught. By using the capture unit 60 illustrated in FIG. 1, eight reaction regions 66 are formed on the surface of the sample analysis disc 70. After drying, the sample analysis disc 70 is analyzed by an analysis device 100 illustrated in FIG. 9, which is an analysis device according to the first and second embodiments.

[Configuration and operation of analysis device]

As illustrated in FIG. 9, the analysis device 100 includes: a turntable 1; a clamper 2; a turntable drive unit 3; a turntable drive circuit 4; an optical pickup drive circuit 5; a reference position detection sensor 6; a guide shaft 7; a pulse detection circuit 8; a controller 9; a storage unit 10; a display 11; and an optical pickup 20. The turntable drive unit 3 can be configured by a spindle motor.

The pulse detection circuit 8 is configured by an integrated circuit which is an FPGA (field programmable gate array) as an example. The pulse detection circuit 8 may be configured by an ASIC (application specific integrated circuit) or a microprocessor. The controller 9 is configured by a CPU (central processing unit). The pulse detection circuit 8 and the controller 9 may be integrated with each other.

The sample analysis disc 70 is mounted on the turntable 1 such that the reaction region 66 faces downward. The clamper 2 is driven in a direction approaching or separating from the turntable 1. When the clamper 2 is driven in the direction approaching the turntable 1 with the sample analysis disc 70 mounted on the turntable 1, the sample analysis disc 70 is held by the turntable 1 and the clamper 2.

The turntable drive unit 3 rotates the turntable 1 around a rotation axis C1 together with the sample analysis disc 70 and the clamper 2. The turntable drive circuit 4 controls the rotation of the turntable 1 by means of the turntable drive unit 3, based on the control by the controller 9. For example, the turntable drive circuit 4 controls the turntable drive unit 3 such that the turntable 1 rotates at a constant linear speed along with the sample analysis disc 70 and the clamper 2.

The reference position detection sensor 6 is arranged near the outer periphery of the sample analysis disc 70. The reference position detection sensor 6 is a reflective optical sensor such as a photoreflector. The reference position detection sensor 6 irradiates the outer periphery of the sample analysis disc 70 with the detection light 6a in a state in which the sample analysis disc 70 rotates, and receives the reflected light from the sample analysis disc 70.

The reference position detection sensor 6 detects the notch 72 of the sample analysis disc 70 to generate a reference position detection signal Sp, and supplies the signal Sp to the controller 9. The reference position detection signal Sp is a pulse signal that becomes low (or high) with respect to every one rotation of the sample analysis disc 70. When the notch 72 of the rotating sample analysis disc 70 is positioned below the reference position detection sensor 6 and the detection light 6a is in a nonreflective state due to the notch 72, the reference position detection signal Sp falls from high to low, and when the notch 72 passes below the reference position detection sensor 6 and the detection light 6a is in a reflective state, the reference position detection signal Sp rises from low to high. A pulse signal having a polarity opposite to the pulse signal generated by the reference position detection sensor 6 may be supplied to the controller 9.

As the reference position detection sensor 6, a photointerrupter which is a transmission type optical sensor may be used. In this case, the reference position detection sensor 6 generates the reference position detection signal Sp which rises from low to high when the detection light 6a passes through the notch 72, and which falls from high to low when the detection light 6a is blocked by the sample analysis disc 70. Even in this case, a pulse signal having a polarity opposite to the pulse signal generated by the reference position detection sensor 6 may be supplied to the controller 9.

The controller 9 detects the reference position for each rotation cycle and track of the sample analysis disc 70, based on the reference position detection signal Sp.

The guide shaft 7 is arranged parallel to the sample analysis disc 70 and along the radial direction of the sample analysis disc 70. The radial direction of the sample analysis disc 70 is a direction orthogonal to the rotation axis C1 of the turntable 1. The optical pickup 20 is supported by the guide axis 7. The optical pickup drive circuit 5 moves the optical pickup 20 along the guide axis 7 in the radial direction of the sample analysis disc 70, based on the control by the controller 9. The optical pickup 20 has an objective lens 21. The optical pickup drive circuit 5 moves the objective lens 21 in a direction approaching or separating from the sample analysis disc 70 in order to perform focus control of the laser beam 20a to be radiated onto the sample analysis disc 70.

The optical pickup drive circuit 5 performs tracking control so as to irradiate the recess 74 of the sample analysis disc 70 with the laser beam 20a. Since the sample analysis disc 70 is rotated by the turntable 1 at a constant linear speed and the laser beam 20a is radiated into the recess 74 under tracking control, the spiral-shaped recess 74 is scanned by the laser beam 20a from the inner periphery to the outer periphery.

The optical pickup 20 receives the reflected light of the laser beam 20a from the sample analysis disc 70. The optical pickup 20 detects the light-reception level of the reflected light to generate a light-reception level signal Sr, and supplies the signal Sr to the pulse detection circuit 8.

Based on the waveform of the light-reception level signal Sr which detects the light-reception level of the reflected light from the reaction region 66, the pulse detection circuit 8 detects a nanoparticle 64 which exists alone and is not close to other nanoparticles 64 within an interference distance within which there is optical interference with other nanoparticles 64, and two or more close nanoparticles 64 which are close to each other within the interference distance. A state in which three or more nanoparticles 64 are close to each other within the interference distance is a state in which two adjacent nanoparticles 64 among such three or more nanoparticles 64 are close to each other within the interference distance. In addition, a state in which two or more nanoparticles 64 are close to each other includes a state in which two or more nanoparticles 64 are in contact with each other.

A nanoparticle 64 which exists alone and is not close to other nanoparticles 64 within the interference distance is called a single nanoparticle. A single nanoparticle may be called a single bead. Close nanoparticles in which two nanoparticles 64 are close to each other within the interference distance are called a double nanoparticle, and close nanoparticles in which three nanoparticles 64 are close to each other within the interference distance are called a triple nanoparticle. Two or more close nanoparticles may be called close beads, and a double nanoparticle and a triple nanoparticle may be called a double bead and a triple bead, respectively.

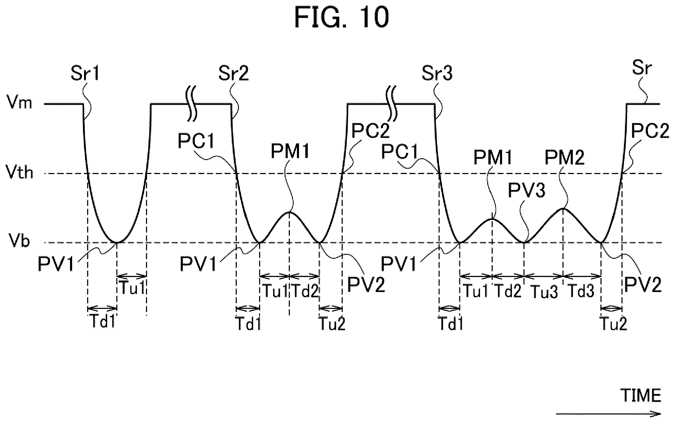

FIG. 10 illustrates an example of the waveforms of the light-reception level signal Sr for a single bead, a double bead, and a triple bead. When the laser beam 20a is not radiated onto a nanoparticle 64, the light-reception level signal Sr is approximately a constant value of a level Vm. When the laser beam 20a is radiated onto a single bead, a single bead waveform Sr1 (a single pulse waveform) that is a downward protruding pulse waveform appears in the light-reception level signal Sr in the amplitude direction. The single bead waveform Sr1 is a waveform in which the level decreases from the level Vm to an extreme value point PV1 which is the position of a minimum value of the amplitude, and then returns to the level Vm. The extreme value point PV1 has a level Vb that is sufficiently lower than a level Vth that is, for example, half the pulse amplitude.

The pulse detection circuit 8 detects a single bead caught in the recess 74, based on the single bead waveform Sr1 appearing in the light-reception level signal Sr. When detecting a single bead, the pulse detection circuit 8 may determine whether it is a single bead by adding the condition that a time Td1 from the level Vth to the extreme value point PV1 and a time Tu1 from the extreme value point PV1 to the level Vth are almost the same time.

When the laser beam 20a is radiated onto a double bead, a double bead waveform Sr2 (two close pulse waveforms) appears in the light-reception level signal Sr. The double bead waveform Sr2 is a waveform in which the level decreases from the level Vm to the extreme value point PV1, then rises to an extreme value point PM1, decreases again to the extreme value point PV2, and then returns to the level Vm. The extreme value point PM1 has a level sufficiently lower than the level Vth. The extreme value point PV2 has the level Vb.

The pulse detection circuit 8 detects the double bead caught in the recess 74, based on the double bead waveform Sr2 appearing in the light-reception level signal Sr. When detecting the double bead, the pulse detection circuit 8 may determine whether it is a double bead by adding the following conditions: the time Td1 from the level Vth to the extreme value point PV1 and the time Tu2 from the extreme value point PV2 to the level Vth are almost the same time; and the time Tu1 from the extreme value point PV1 to the extreme value point PM1 and the time Td2 from the extreme value point PM1 to the extreme value point PV2 are almost the same time.

When the laser beam 20a is radiated onto a triple bead, a triple bead waveform Sr3 (three close pulse waveforms) appears in the light-reception level signal Sr. The triple bead waveform Sr3 is a waveform in which the level decreases from the level Vm to the extreme value point PV1, then rises to the extreme value point PM1, decreases again to the extreme value point PV3, further rises to the extreme value point PM2, decreases to the extreme value point PV2, and then returns to the level Vm. The extreme value points PM1 and PM2 have levels sufficiently lower than the level Vth. The extreme value point PV3 has the level Vb.

The pulse detection circuit 8 detects the triple bead caught in the recess 74, based on the triple bead waveform Sr3 appearing in the light-reception level signal Sr. When detecting the triple bead, the pulse detection circuit 8 may determine whether it is a triple bead by adding the following conditions: the time Td1 from the level Vth to the extreme value point PV1 and the time Tu2 from the extreme value point PV2 to the level Vth are almost the same time; the time Tu1 from the extreme value point PV1 to the extreme value point PM1 and the time Td2 from the extreme value point PM1 to the extreme value point PV3 are almost the same time; and the time Tu3 from the extreme value point PV3 to the extreme value point PM2 and the time Td3 from the extreme value point PM2 to the extreme value point PV2 are almost the same time.

The pulse detection circuit 8 may detect a quadruple bead or more than four close beads by using a detection method similar to that for detecting a double or a triple bead. Where n is an integer greater than or equal to 2, the pulse detection circuit 8 detects at most n-beads. The pulse detection circuit 8 detects (n - 1) kinds of close pulse-like waveforms, and distinguishes a single pulse waveform and (n - 1) kinds of close pulse-like waveforms from each other. In reality, since there is almost no quadruple or a close bead more than that, it is sufficient for the pulse detection circuit 8 to detect single beads, double beads and triple beads defining a maximum number n of 3.

The pulse detection circuit 8 supplies each detection value from a single bead to n-beads, to the controller 9. In a case where n is set to 3, the pulse detection circuit 8 supplies a first detection value indicating that a single bead has been detected if a single bead has been detected, a second detection value indicating that a double bead has been detected if a double bead has been detected, and a third detection value indicating that a triple bead has been detected if a triple bead has been detected, to the controller 9 when each track in each reaction region 66 is scanned by the laser beam 20a.

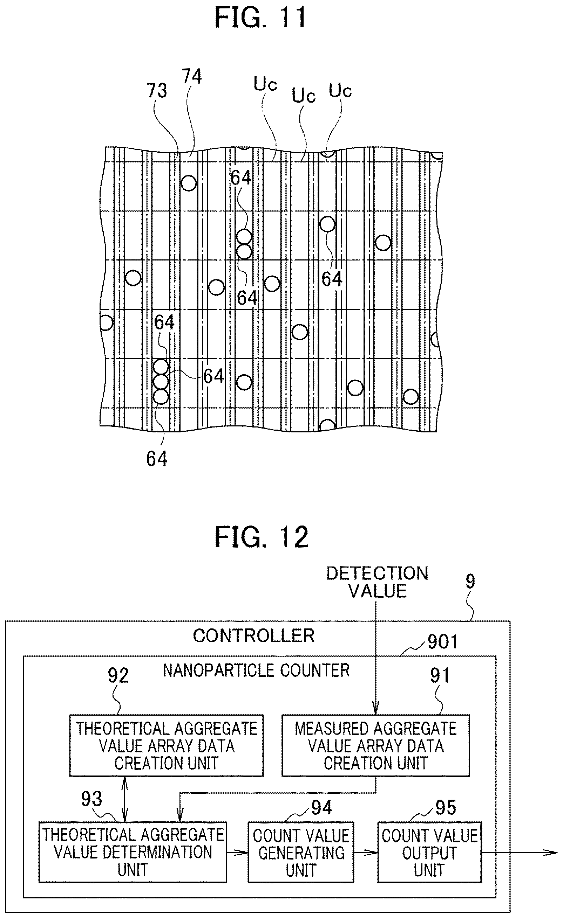

As illustrated in FIG. 11, the controller 9 divides the reaction region 66 into a plurality of mesh-like unit sections Uc, and obtains a detection value supplied from the pulse detection circuit 8 for each unit section Uc. Since the controller 9 recognizes the circumferential position in the reaction region 66 on the basis of the reference position detection signal Sp and recognizes the track scanned by the laser beam 20a by means of the tracking control performed by the optical pickup drive circuit 5, the reaction region 66 can be divided into the plurality of unit sections Uc.

In the example illustrated in FIG. 11, the unit section Uc has a length in the radial direction including the width of the recess 74 of one track, and a length in the circumferential direction that is long enough to accommodate a triple bead. However, the unit section Uc is not limited to the above sizes, and may have a length in the radial direction including the width of the recess 74 of two tracks, or a length in the circumferential direction that is long enough to accommodate a quadruple bead or more than four close beads.

[Analysis device and analysis method according to first embodiment]

An object of the first embodiment is to provide an analysis device and an analysis method capable of, compared to the past, more correctly counting single beads and double beads to n-beads, and further avoiding the influence of aggregation of the nanoparticles 64, which label the detection target substance 63, and assay noise.

As illustrated in FIG. 12, the controller 9 of the analysis device 100 according to the first embodiment includes a nanoparticle counter 901 as a functional configuration. The nanoparticle counter 901 includes; a measured aggregate value array data creation unit 91; a theoretical aggregate value array data creation unit 92; a theoretical aggregate value determination unit 93; a count value generating unit 94; and a count value output unit 95.

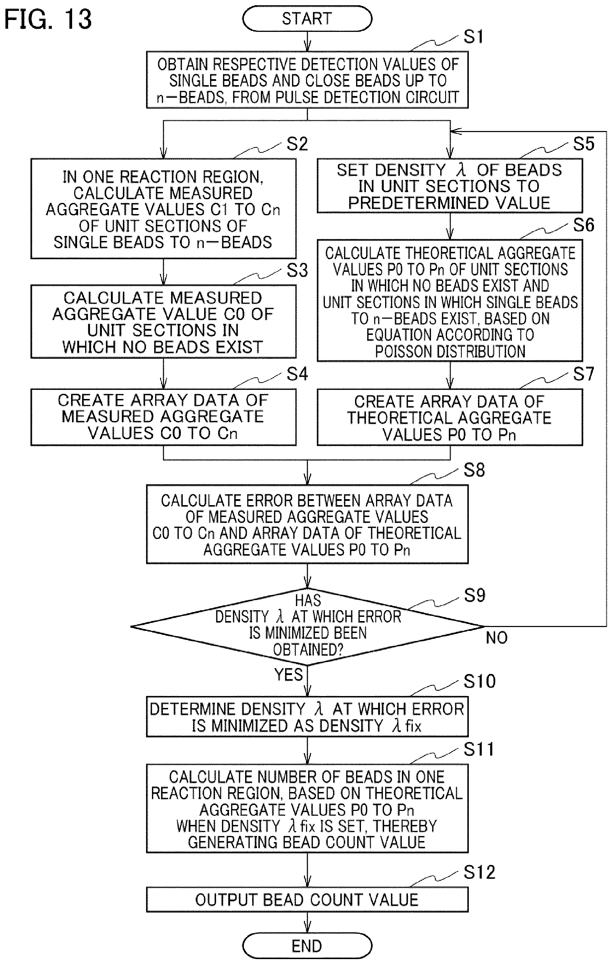

Specific processing executed by the controller 9 (the nanoparticle counter 901) will be described with reference to the flowchart illustrated in FIG. 13. When the controller 9 starts the processing, the controller 9 obtains from the pulse detection circuit 8 in step S1, respective detection values of single beads and close beads up to n-beads. In step S2, in one reaction region 66, the controller 9 calculates measured aggregate values C1 to Cn of the unit section Uc from single beads to n-beads.

The measured aggregate value C1 is the number of unit sections Uc in which single beads are detected out of the number M of all unit sections Uc in one reaction region 66. The measured aggregate value C2 is the number of unit sections Uc in which double beads are detected out of the number M of unit sections Uc. The measured aggregate value C3 is the number of unit sections Uc in which triple beads are detected out of the number M of unit sections Uc. In this way, the controller 9 individually aggregates the number of unit sections Uc, from the unit sections Uc in which single beads are detected to the unit sections Uc in which n-beads are detected.

In step S3, the controller 9 calculates a measured aggregate value C0 of the unit sections Uc in which no beads (nanoparticles 64) exist. The measured aggregate value C0 is the number of unit sections Uc in which no nanoparticles 64 exist, out of the number M of unit sections Uc. When k is a variable from 1 to n, the measured aggregate value C0 is obtained by $M-\Sigma(Ck)$ which subtracts the sum of the measured aggregate values C1 to Cn from the number M. In step S4, the controller 9 creates array data (first array data) of the measured aggregate values C0 to Cn. The processing in steps S1 to S4 is executed by the measured aggregate value array data creation unit 91.

The controller 9 executes the processing of steps S5 to S7 in parallel with that of steps S2 to S4. In step S5, the controller 9 sets a density $\lambda$ of the beads (nanoparticles 64) in the unit section Uc to a predetermined value. In step S6, the controller 9 calculates the theoretical aggregate values P0 to Pn of the unit section Uc in which no beads exist and the unit section Uc in which single beads to n-beads exist, based on the probability distribution according to the theory of Poisson distribution. The theory of Poisson distribution is a typical example of a probability theory.

Specifically, the controller 9 calculates the theoretical aggregate values P0 to Pn, based on the following equation (1) according to the theory of Poisson distribution. Here, k is a variable from 0 to n, and e is the base of a natural logarithm (Napier number).

$$Pk(\lambda) = M \times \lambda^k \times e^{-\lambda} / k! \ ...(1)$$

In step S7, the controller 9 creates array data (second array data) of the theoretical aggregate values P0 to Pn. The processing in steps S5 to S7 is executed by the theoretical aggregate value array data creation unit 92.

In step S8, the controller 9 calculates an error between the array data of the measured aggregate values C0 to Cn and the array data of the theoretical aggregate values P0 to Pn. As an example, the controller 9 quantifies a difference Diff using the following equation (2).

$$Diff = (\Sigma_k(|Pk(\lambda) - Ck|^2))^{1/2} \ ...(2)$$

In step S9, the controller 9 determines whether the density $\lambda$ at which the difference Diff is minimized has been obtained. The processing in steps S8 and S9 is executed by the theoretical aggregate value determination unit 93. If the density $\lambda$ at which the difference Diff is minimized is not obtained (NO), the controller 9 returns the processing to step S5. The density $\lambda$ at which the difference Diff is minimized cannot be obtained by executing the processing in step S8 only once.

Thus, the controller 9 sets a new value of the density $\lambda$ in step S5, and executes the processing in steps S5 to S8 again. For example, the controller 9 may first set the density $\lambda$ to a very small value that is close to 0, and gradually increase the value of the density $\lambda$. If the density $\lambda$ at which the difference Diff is minimized is not obtained, the theoretical aggregate value determination unit 93 controls the theoretical aggregate value array data creation unit 92 to calculate the theoretical aggregate values P0 to Pn by setting the density $\lambda$ to a new value and using the new value of the density $\lambda$.

The controller 9 repeats the processing of steps S5 to S8 by updating the value of the density $\lambda$, thereby obtaining the density $\lambda$ at which the difference Diff is minimized. In step S9, if the density $\lambda$ at which the difference Diff is minimized is obtained (YES), the controller 9 determines the density $\lambda$ at which the difference Diff is minimized as a density $\lambda$fix in step S10. The processing in step S10 is executed by the theoretical aggregate value determination unit 93.

In step S11, the controller 9 calculates the number of beads (nanoparticles 64) in one reaction region 66 (aggregate number), based on the theoretical aggregate values P0 to Pn when the density $\lambda$fix is set, thereby generating a bead count value Vbc. The processing in step S11 is executed by the count value generating unit 94.

The bead count value Vbc is represented by the following equation (3). Since the theoretical aggregate value P0 is a theoretical aggregate value of the unit section Uc in which no nanoparticles 64 exist, the bead count value Vbc obtained by the following equation (3) is equivalent to an aggregate number based on the theoretical aggregate values P1 to Pn.

$$Vbc = \Sigma_k(k \times Pk(\lambda fix)) \ ...(3)$$

In step S12, the controller 9 outputs the bead count value Vbc to terminate the processing. The processing in step S12 is executed by the count value output unit 95. The controller 9 supplies the bead count value Vbc to the storage unit 10 to be stored in the storage unit 10. Although not illustrated in FIG. 13, the controller 9 displays the bead count value Vbc on the display 11 such that the operator can check the bead count value Vbc.

FIG. 13 illustrates processing of the generation and output of an bead count value Vbc in one reaction region 66. When a plurality of reaction regions 66 are formed on the sample analysis disc 70, the controller 9 executes the processing illustrated in FIG. 13 for each reaction region 66. The storage unit 10 stores the bead count value Vbc for each reaction region 66. The display 11 displays the bead count value Vbc for each reaction region 66.

Next, a specific example will be described. In a case where the diameter of the well 61 was 6 mm, where the size of the unit section Uc was 320 nm × 600 nm, and where the number M of the unit sections Uc in one reaction region 66 was $1.47 \times 10^8$, when the measured aggregate values C0 to Cn in one reaction region 66 were calculated, the following values were obtained. The measured aggregate value C0 was the $9.1 \times 10^7$, the measured aggregate value C1 was $3.2 \times 10^7$, the measured aggregate value C2 was $1.8 \times 10^7$, and the measured aggregate value C3 was $6.9 \times 10^6$.

The density λfix at which the difference Diff was minimized was obtained as 0.4566. Regarding the theoretical aggregate values P0 to Pn obtained at this time, the theoretical aggregate value P0 was $9.1 \times 10^7$, the theoretical aggregate value P1 was $3.2 \times 10^7$, the theoretical aggregate value P2 was $1.8 \times 10^7$, and the theoretical aggregate value P3 was $6.9 \times 10^6$.

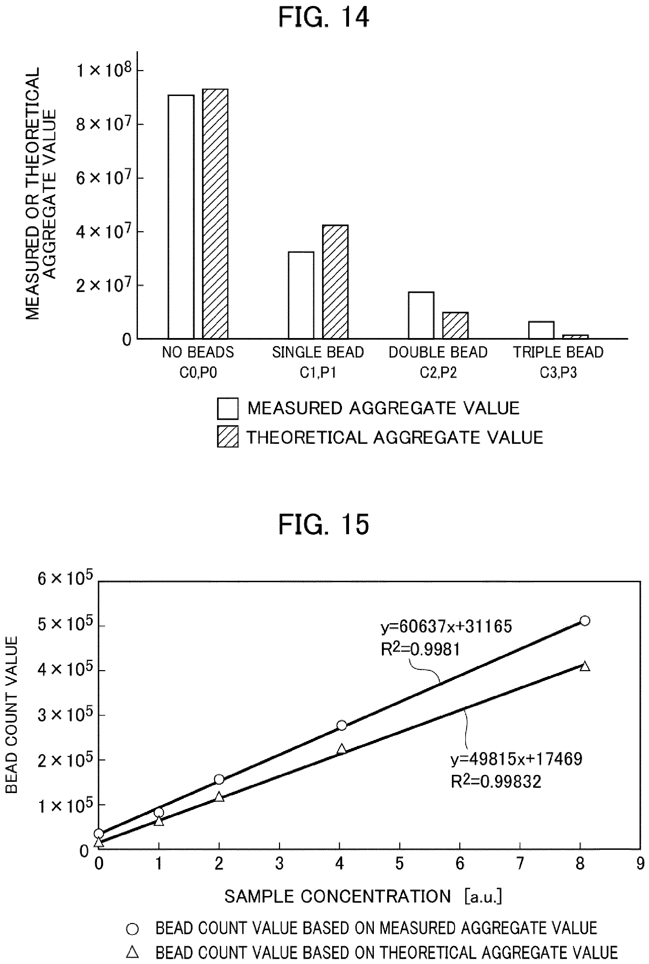

Thus, the array data of the measured aggregate values C0 to Cn and the theoretical aggregate values P0 to Pn are as illustrated in FIG. 14. At this time, the difference Diff based on the equation (2) is 14489226.89. As illustrated in FIG. 14, as for single beads, the measured aggregate value C1 is less than the theoretical aggregate value P1, and as for double beads and triple beads, the measured aggregate values C2 and C3 are greater than the theoretical aggregate values P2 and P3, respectively.

It is conceivable that, in the double beads and the triple beads, the measured aggregate values C2 and C3 are greater than the theoretical aggregate values P2 and P3, respectively, due to the influence of aggregation of the nanoparticles 64 and assay noise. In the single beads, the measured aggregate value C1 is less than the theoretical aggregate value P1 because double beads or triple beads were detected in the unit section Uc where a single bead should have been detected.

Instead of generating the bead count value Vbc on the basis of the measured aggregate values C1 to Cn, the controller 9 (the aggregate value generating unit 94) generates the bead count value Vbc on the basis of the theoretical aggregate values P1 to Pn in the theoretical aggregate values P0 to Pn that most closely approximate the measured aggregate values C0 to Cn. Therefore, the analysis device 100 makes it possible to, compared to the past, more correctly count the nanoparticles 64 fixed to the reaction region 66, and further to avoid the influence of aggregation of the nanoparticles 64, which label the detection target substance 63, and assay noise.

The analysis device 100 makes it possible to, compared to the past, more correctly count a nanoparticle 64 which exists alone and is not close to other nanoparticles 64 within an interference distance within which there is optical interference with other nanoparticles 64, and two or more close nanoparticles 64 which are close to each other within the interference distance.

FIG. 15 illustrates the bead count value Vbc based on the measured aggregate values C1 to Cn and the bead count value Vbc based on the theoretical aggregate values P1 to Pn which are obtained at each concentration of 0, 1, 2, 4, and 8 when the concentration (sample concentration) of the detection target substance 63 in the sample solution to be injected into the well 61 increases to each of the above concentrations 0, 1, 2, 4, and 8. The unit of concentration is a.u. A concentration of 0 is a state that contains only dilute solution and no detectable substance 63.

The sample concentration illustrated in FIG. 15 is about 1/100 of the sample concentration in the above specific example. For this reason, the bead count value Vbc is much smaller than the bead count value Vbc calculated in the above specific example.

When the sample concentration on the horizontal axis is x and the bead count value Vbc on the vertical axis is y, the approximate straight line of the bead count value Vbc obtained based on the measured aggregate values C1 to Cn is represented by the following equation (4). The approximate straight line of the bead count value Vbc obtained based on the theoretical aggregate values P1 to Pn is represented by the following equation (5).

$$y = 60637x + 31165 \ ...(4)$$

$$y = 49815x + 17469 \ ...(5)$$

The value $R^2$ indicating the linearity of the approximate straight line represented by the equation (4) is 0.9981, and the value $R^2$ indicating the linearity of the approximate straight line represented by equation (5) is 0.9983. It is preferable that the value $R^2$ indicating the linearity be close to 1. The controller 9 generates the bead count value Vbc based on the theoretical aggregate values P1 to Pn instead of the measured aggregate values C1 to Cn, thereby improving the linearity obtained when changing a sample concentration.

The bead count value Vbc obtained when the concentration is 0 indicates background noise. In FIG. 15, when the bead count value Vbc is generated based on the theoretical aggregate values P1 to Pn, the background noise is reduced by about 56% compared to when the bead count value Vbc is generated based on the measured aggregate values C1 to Cn. The slope of the approximate line represented by the equation (5) is reduced to 82% compared to the slope of the approximate line represented by the equation (4). However, since the background noise is greatly reduced, the analysis device 100 has a good S/N ratio and is not susceptible to noise.

[Analysis device and analysis method according to second embodiment]

An object of the second embodiment is to provide an analysis device and an analysis method capable of quantitatively evaluating the degree of aggregation of the nanoparticles 64 which label the detection target substance 63.

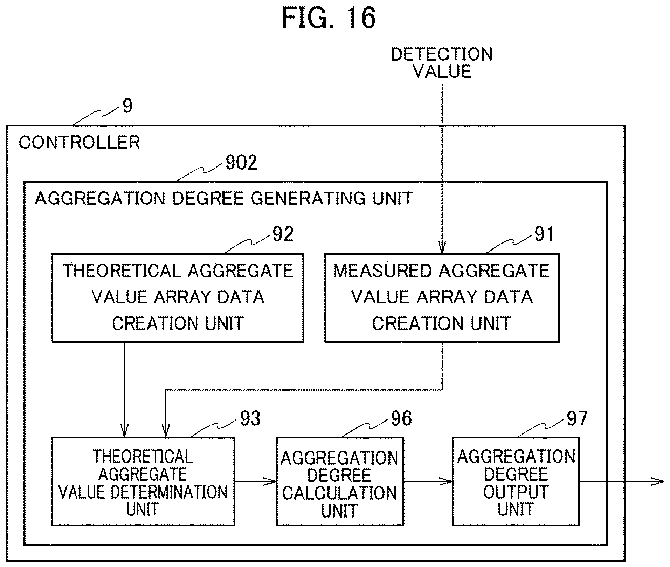

As illustrated in FIG. 16, the controller 9 in the analysis device 100 according to the second embodiment includes an aggregation degree generating unit 902 as a functional structure. In the aggregation degree generating unit 902, the same parts as those of the nanoparticle counter 901 are given the same reference numerals, and the description thereof will be omitted in some cases. The aggregation degree generating unit 902 includes an aggregation degree calculation unit 96 and an aggregation degree output unit 97 instead of the count value generating unit 94 and the count value output unit 95.

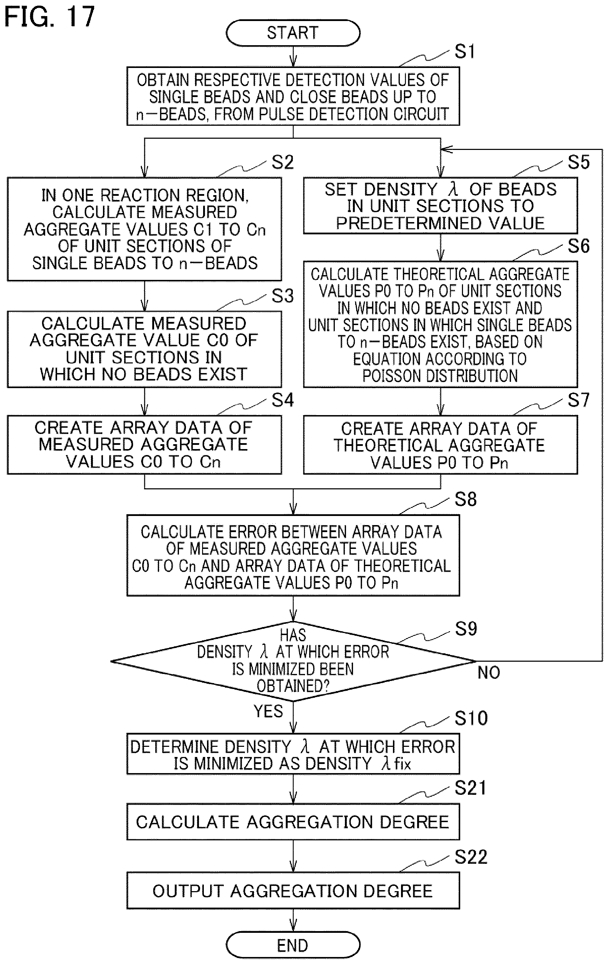

Specific processing executed by the controller 9 (the aggregation degree generating unit 902) will be described with reference to a flowchart illustrated in FIG. 17. In FIG. 17, the same parts as those of the processing illustrated in FIG. 13 are denoted by the same reference numerals, and the description thereof will be omitted in some cases.

After the controller 9 (the aggregation degree calculation unit 96) determines in step S10 that the density $\lambda$ at which the difference Diff is minimized is the density $\lambda$fix, the controller 9 calculates an aggregation degree that indicates the degree of aggregation of the nanoparticles 64 in step S21. The aggregation degree calculation unit 96 calculates an aggregation degree Agg that indicates the degree of aggregation of the nanoparticles 64 using the following equation (6). In the following equation (6), Diff($\lambda$fix) is the difference Diff at the density $\lambda$fix.

$$\text{Agg} = \text{Diff}(\lambda \text{fix}) \; / \; \Sigma_k(k \times Ck)$$
$$= (\Sigma_k(|Pk(\lambda \text{fix}) - Ck|^2))^{1/2} \; / \; \Sigma_k(k \times Ck) \; ...(6)$$

As can be seen from the above equation (6), the aggregation degree Agg is a value obtained by dividing a quantitative difference Diff($\lambda$fix) between the array data of the measured aggregate values C0 to Cn and the array data of the theoretical aggregate values P0 to Pn by the count value of the nanoparticles 64 based on the measured aggregate values C0 to Cn. The count value of the nanoparticles 64 based on the measured aggregate values C0 to Cn is a count value obtained by calculating the number of nanoparticles 64 in the reaction region 66, based on the measured aggregate value C1 of the unit sections where single beads exist and the measured aggregate values C2 to Cn of the unit sections where 2 to n close beads exist.

In step S22, the controller 9 (the aggregation degree output unit 97) outputs the aggregation degree Agg to terminate the processing. The controller 9 supplies the aggregation degree Agg to the storage unit 10 to be stored in the storage unit 10. Although not illustrated in FIG. 17, the controller 9 displays the aggregation degree Agg on the display 11 such that the operator can check the aggregation degree Agg.

FIG. 17 illustrates processing of the generation and output of the aggregation degree Agg in one reaction region 66. When a plurality of reaction regions 66 are formed in the sample analysis disc 70, the controller 9 executes the processing illustrated in FIG. 17 for each reaction region 66. The storage unit 10 stores the aggregation degree Agg for each reaction region 66. The display 11 displays the aggregation degree Agg for each reaction region 66.

In the specific example illustrated in FIG. 14, when the aggregation degree Agg is calculated based on the equation (6), the aggregation degree Agg becomes 0.1695.

Meanwhile, the value of the aggregation degree Agg varies depending on the variation in the process of preparing the nanoparticles 64 in which the antibodies 65 are fixed onto the surface. That is, the value of the aggregation degree Agg may vary depending on the lot when preparing the nanoparticles 64. For this reason, the controller 9 may store the aggregation degree Agg in the storage unit 10 corresponding to a lot identification number identifying a lot which is issued when preparing the nanoparticles 64. The display 11 may display the aggregation degree Agg corresponding to a lot identification number.

This makes it possible to improve the process of preparing the nanoparticles 64 so as to reduce the aggregation degree Agg as much as possible. In addition, when there are multiple places where the nanoparticles 64 can be purchased, the purchaser can select a place that prepares the nanoparticles 64 having a small aggregation degree Agg.

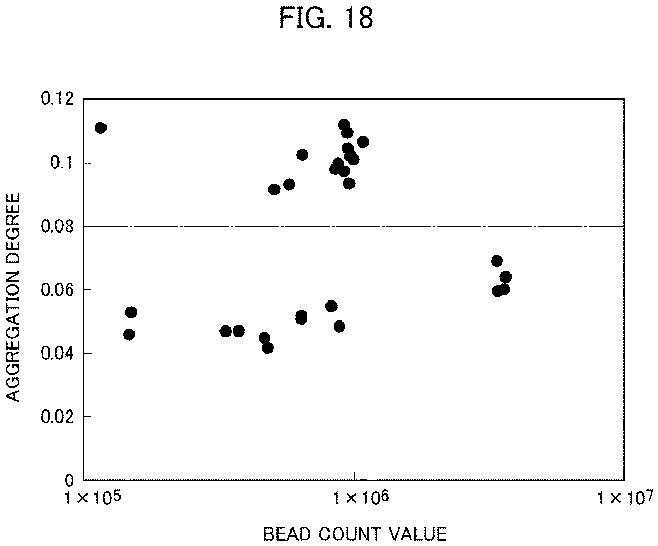

FIG. 18 illustrates an example of plotting the relationship between the bead count Vbc and the aggregation degree Agg obtained when the sample analysis disc 70 is analyzed using nanoparticles 64 of different lots, with the bead count value Vbc on the horizontal axis and the aggregation degree Agg on the vertical axis. For example, it is determined that if the aggregation degree Agg is 0.08 or less, the aggregation degree Agg is good, and that if the aggregation degree Agg exceeds 0.08, the aggregation degree Agg is not good.

The controller 9 may store the determination result as to whether the aggregation degree Agg is good in the storage unit 10. The determination result may be a value of "0" if the aggregation degree Agg is good, and a value of "1" if the aggregation degree Agg is not good. The controller 9 may display the determination result on the display 11 on the basis of a determination value as to whether the aggregation degree Agg is good.

[Analysis device and analysis method according to third embodiment]

An object of the third embodiment is to achieve both the object of the first embodiment and the object of the second embodiment.

The controller 9 includes the aggregation degree calculation unit 96 and the aggregation degree output unit 97 in conjunction with the count value generating unit 94 and the count value output unit 95. That is, the analysis device 100 according to the third embodiment includes both the nanoparticle counter 901 of the first embodiment and the aggregation degree generating unit 902 of the second embodiment. The analysis device 100 according to the third embodiment stores the bead count value Vbc and the aggregation degree Agg in the storage unit 10 and displays such values on the display 11. The analysis method according to the third embodiment analyzes the sample analysis disc 70 on the basis of the bead count value Vbc and the aggregation degree Agg.

The present invention is not limited to the first to the third embodiments described above, and may be varied in various ways without departing from the scope of the present invention.

In the structures of FIGS. 12 and 16, the controller 9 (the theoretical aggregate value array data creation unit 92) calculates the theoretical aggregate values P0 to Pn to create array data. A plurality of sets of array data of the theoretical aggregate values P0 to Pn obtained when differentiating the value of density $\lambda$ may be created in advance and stored in the storage unit 10. In this case, the theoretical aggregate value determination unit 93 selects the array data of the theoretical aggregate values P0 to Pn that most closely approximates the array data of the measured aggregate values C0 to Cn, from among the plurality of sets of array data read from the storage unit 10.

This application claims priority under Japanese Patent Application Nos. 2021-044920 and 2021-044933 filed with the Japan Patent Office on March 18, 2021, the entire contents of both of which are incorporated herein by reference.

**Claims**

1. An analysis device comprising:

   an optical pickup configured to irradiate a sample analysis disc with a laser beam and to detect a light-reception level of reflected light from a reaction region to generate a light-reception level signal, the sample analysis disc having the reaction region, and the reaction region having fixed thereto a plurality of detection target substances having nanoparticles that are labels bound thereto;
   a pulse detection circuit configured to detect, from a waveform of the light-reception level signal, a single pulse waveform indicating a single nanoparticle that exists alone, and (n - 1) types of close pulse waveforms indicating 2 to n close nanoparticles (n being an integer) in which adjacent nanoparticles are close to each other within an interference distance, thereby generating respective detection values; and
   a nanoparticle counter configured to generate a count value of the nanoparticles in the reaction region,
   wherein the nanoparticle counter:

   divides the reaction region into a plurality of unit sections;
   based on the respective detection values, individually aggregates the number of unit sections in which the single pulse waveform is detected, and the number of unit sections in which the close pulse waveforms are detected with respect to each of the (n - 1) types, and generates a measured aggregation value of unit sections in which the single nanoparticle exists alone and respective measured aggregation values of unit sections in which the 2 to n close nanoparticles exist;
   calculates the number of unit sections in which neither of the single pulse waveform and the close pulse waveforms are detected by subtracting, from the number of all unit sections in the reaction region, the number of unit sections in which the single pulse waveform is detected and the number of unit sections in which the close pulse waveforms are detected with respect to each of the (n - 1) types, thereby generating a measured aggregate value of unit sections in which no nanoparticles exist;
   generates first array data of the measured aggregate value of unit sections in which no nanoparticles exist, the measured aggregation value of unit sections in which the single nanoparticle exists, and the measured aggregation values of unit sections in which the 2 to n close nanoparticles exist;

generates or selects second array data of a theoretical aggregate value of unit sections in which no nanoparticles exist, a theoretical aggregation value of unit sections in which the single nanoparticle exists, and theoretical aggregation values of unit sections in which the 2 to n close nanoparticles exist, the second array data being array data of theoretical aggregate values based on a probability distribution according to a probability theory and most closely approximating the first array data; and

calculates the number of nanoparticles in the reaction region, based on the theoretical aggregation value of unit sections in which the single nanoparticle exists and the theoretical aggregation values of unit sections in which the 2 to n close nanoparticles exist.

2. An analysis device comprising:

an optical pickup configured to irradiate a sample analysis disc with a laser beam and to detect a light-reception level of reflected light from a reaction region to generate a light-reception level signal, the sample analysis disc having the reaction region, and the reaction region having fixed thereto a plurality of detection target substances having nanoparticles that are labels bound thereto;

a pulse detection circuit configured to detect, from a waveform of the light-reception level signal, a single pulse waveform indicating a single nanoparticle that exists alone, and (n - 1) types of close pulse waveforms indicating 2 to n close nanoparticles (n being an integer) in which adjacent nanoparticles are close to each other within an interference distance, thereby generating respective detection values; and

an aggregation degree generating unit configured to calculate an aggregation degree indicating a degree of aggregation of the nanoparticles in the reaction region,

wherein the aggregation degree generating unit:

divides the reaction region into a plurality of unit sections;

based on the respective detection values, individually aggregates the number of unit sections in which the single pulse waveform is detected, and the number of unit sections in which the close pulse waveforms are detected with respect to each of the (n - 1) types, and generates a measured aggregation value of unit sections in which the single nanoparticle exists alone and respective measured aggregation values of unit sections in which the 2 to n close nanoparticles exist;

calculates the number of unit sections in which neither of the single pulse waveform and the close pulse waveforms are detected by subtracting, from the number of all unit sections in the reaction region, the number of unit sections in which the single pulse waveform is detected and the number of unit sections in which the close pulse waveforms are detected with respect to each of the (n - 1) types, thereby generating a measured aggregate value of unit sections in which no nanoparticles exist;

generates first array data of the measured aggregate value of unit sections in which no nanoparticles exist, the measured aggregation value of unit sections in which the single nanoparticle exists, and the measured aggregation values of unit sections in which the 2 to n close nanoparticles exist;

generates or selects second array data of a theoretical aggregate value of unit sections in which no nanoparticles exist, a theoretical aggregation value of unit sections in which the single nanoparticle exists, and theoretical aggregation values of unit sections in which the 2 to n close nanoparticles exist, the second array data being array data of theoretical aggregate values based on a probability distribution according to a probability theory and most closely approximating the first array data; and

divides a quantitative difference between the first array data and the second array data by a count value obtained by calculating the number of nanoparticles in the reaction region, based on the measured aggregation value of unit sections in which the single nanoparticle exists and the measured aggregation values of unit sections in which the 2 to n close nanoparticles exist.

3. The analysis device according to claim 1 or 2, wherein a Poisson distribution is used for the probability distribution when calculating the theoretical aggregation values.

4. The analysis device according to claim 1, wherein the nanoparticle counter divides the reaction region into unit sections that are large enough to accommodate three close nanoparticles.

5. An analysis method comprising:

irradiating a sample analysis disc with a laser beam by means of an optical pickup, the sample analysis disc having a reaction region to which there are fixed a plurality of detection target substances having nanoparticles that are labels bound thereto;

detecting a light-reception level of reflected light from the reaction region and generating a light-reception level signal by means of the optical pickup; and

detecting, from a waveform of the light-reception level signal, a single pulse waveform indicating a single nanoparticle that exists alone, and (n - 1) types of close pulse waveforms indicating 2 to n close nanoparticles (n being an integer) in which adjacent nanoparticles are close to each other within an interference distance, thereby generating respective detection values, by means of a pulse detection circuit;

wherein the analysis method includes, by means of a controller that obtains the respective detection values, dividing the reaction region into a plurality of unit sections;

based on the respective detection values, individually aggregating the number of unit sections in which the single pulse waveform is detected, and the number of unit sections in which the close pulse waveforms are detected with respect to each of the (n - 1) types, and generating a measured aggregation value of unit sections in which the single nanoparticle exists alone and respective measured aggregation values of unit sections in which the 2 to n close nanoparticles exist;

calculating the number of unit sections in which neither of the single pulse waveform and the close pulse waveforms are detected by subtracting, from the number of all unit sections in the reaction region, the number of unit sections in which the single pulse waveform is detected and the number of unit sections in which the close pulse waveforms are detected with respect to each of the (n - 1) types, thereby generating a measured aggregate value of unit sections in which no nanoparticles exist;

generating first array data of the measured aggregate value of unit sections in which no nanoparticles exist, the measured aggregation value of unit sections in which the single nanoparticle exists, and the measured aggregation values of unit sections in which the 2 to n close nanoparticles exist;

generating or selecting second array data of a theoretical aggregate value of unit sections in which no nanoparticles exist, a theoretical aggregation value of unit sections in which the single nanoparticle exists, and theoretical aggregation values of unit sections in which the 2 to n close nanoparticles exist, the second array data being array data of theoretical aggregate values based on a probability distribution according to a probability theory and most closely approximating the first array data; and

calculating the number of nanoparticles in the reaction region, based on the theoretical aggregation value of unit sections in which the single nanoparticle exists and the theoretical aggregation values of unit sections in which the 2 to n close nanoparticles exist, thereby generating a count value of the nanoparticles in the reaction region.

6. An analysis method comprising:

irradiating a sample analysis disc with a laser beam by means of an optical pickup, the sample analysis disc having a reaction region to which there are fixed a plurality of detection target substances having nanoparticles that are labels bound thereto;

detecting a light-reception level of reflected light from the reaction region and generating a light-reception level signal by means of the optical pickup; and

detecting, from a waveform of the light-reception level signal, a single pulse waveform indicating a single nanoparticle that exists alone, and (n - 1) types of close pulse waveforms indicating 2 to n close nanoparticles (n being an integer) in which adjacent nanoparticles are close to each other within an interference distance, thereby generating respective detection values, by means of a pulse detection circuit;

wherein the analysis method includes, by means of a controller that obtains the respective detection values, dividing the reaction region into a plurality of unit sections;

based on the respective detection values, individually aggregating the number of unit sections in which the single pulse waveform is detected, and the number of unit sections in which the close pulse waveforms are detected with respect to each of the (n - 1) types, and generating a measured aggregation value of unit sections in which the single nanoparticle exists alone and respective measured aggregation values of unit sections in which the 2 to n close nanoparticles exist;

calculating the number of unit sections in which neither of the single pulse waveform and the close pulse waveforms are detected by subtracting, from the number of all unit sections in the reaction region, the number of unit sections in which the single pulse waveform is detected and the number of unit sections in which the close pulse waveforms are detected with respect to each of the (n - 1) types, thereby generating a measured aggregate value of unit sections in which no nanoparticles exist;

generating first array data of the measured aggregate value of unit sections in which no nanoparticles exist, the measured aggregation value of unit sections in which the single nanoparticle exists, and the measured aggregation values of unit sections in which the 2 to n close nanoparticles exist;

generating or selecting second array data of a theoretical aggregate value of unit sections in which no nanoparticles exist, a theoretical aggregation value of unit sections in which the single nanoparticle exists, and theoretical aggregation values of unit sections in which the 2 to n close nanoparticles exist, the second array data being array data of theoretical aggregate values based on a probability distribution according to a probability theory and most closely approximating the first array data; and

dividing a quantitative difference between the first array data and the second array data by a count value obtained by calculating the number of nanoparticles in the reaction region, based on the measured aggregation value of unit sections in which the single nanoparticle exists and the measured aggregation values of unit sections in which the 2 to n close nanoparticles exist, thereby calculating an aggregation degree indicating a degree of aggregation of the nanoparticles in the reaction region.

FIG. 1

FIG. 2

FIG. 3

FIG. 4

# FIG. 5

# FIG. 6

# FIG. 7A

# FIG. 7B

# FIG. 7C

FIG. 8

# FIG. 9

EP 4 310 497 A1

## FIG. 10

# FIG. 11

# FIG. 12

## FIG. 13

```
                          START

                            │ S1
                            ▼
  OBTAIN RESPECTIVE DETECTION VALUES OF
     SINGLE BEADS AND CLOSE BEADS UP TO
  n−BEADS, FROM PULSE DETECTION CIRCUIT
```

S2 — IN ONE REACTION REGION, CALCULATE MEASURED AGGREGATE VALUES C1 TO Cn OF UNIT SECTIONS OF SINGLE BEADS TO n−BEADS

S3 — CALCULATE MEASURED AGGREGATE VALUE C0 OF UNIT SECTIONS IN WHICH NO BEADS EXIST

S4 — CREATE ARRAY DATA OF MEASURED AGGREGATE VALUES C0 TO Cn

S5 — SET DENSITY λ OF BEADS IN UNIT SECTIONS TO PREDETERMINED VALUE

S6 — CALCULATE THEORETICAL AGGREGATE VALUES P0 TO Pn OF UNIT SECTIONS IN WHICH NO BEADS EXIST AND UNIT SECTIONS IN WHICH SINGLE BEADS TO n−BEADS EXIST, BASED ON EQUATION ACCORDING TO POISSON DISTRIBUTION

S7 — CREATE ARRAY DATA OF THEORETICAL AGGREGATE VALUES P0 TO Pn

S8 — CALCULATE ERROR BETWEEN ARRAY DATA OF MEASURED AGGREGATE VALUES C0 TO Cn AND ARRAY DATA OF THEORETICAL AGGREGATE VALUES P0 TO Pn

S9 — HAS DENSITY λ AT WHICH ERROR IS MINIMIZED BEEN OBTAINED? — NO / YES

S10 — DETERMINE DENSITY λ AT WHICH ERROR IS MINIMIZED AS DENSITY λfix

S11 — CALCULATE NUMBER OF BEADS IN ONE REACTION REGION, BASED ON THEORETICAL AGGREGATE VALUES P0 TO Pn WHEN DENSITY λfix IS SET, THEREBY GENERATING BEAD COUNT VALUE

S12 — OUTPUT BEAD COUNT VALUE

END

## FIG. 14

MEASURED OR THEORETICAL AGGREGATE VALUE (y-axis)

$1 \times 10^8$
$8 \times 10^7$
$6 \times 10^7$
$4 \times 10^7$
$2 \times 10^7$
$0$

NO BEADS C0,P0     SINGLE BEAD C1,P1     DOUBLE BEAD C2,P2     TRIPLE BEAD C3,P3

☐ MEASURED AGGREGATE VALUE
▨ THEORETICAL AGGREGATE VALUE

## FIG. 15

BEAD COUNT VALUE (y-axis)

$6 \times 10^5$
$5 \times 10^5$
$4 \times 10^5$
$3 \times 10^5$
$2 \times 10^5$
$1 \times 10^5$
$0$

$y=60637x+31165$
$R^2=0.9981$

$y=49815x+17469$
$R^2=0.99832$

SAMPLE CONCENTRATION [a.u.]

○ BEAD COUNT VALUE BASED ON MEASURED AGGREGATE VALUE
△ BEAD COUNT VALUE BASED ON THEORETICAL AGGREGATE VALUE

# FIG. 16

DETECTION
VALUE

9

CONTROLLER

902

AGGREGATION DEGREE GENERATING UNIT

| 92 | 91 |
|---|---|
| THEORETICAL AGGREGATE VALUE ARRAY DATA CREATION UNIT | MEASURED AGGREGATE VALUE ARRAY DATA CREATION UNIT |

93

THEORETICAL AGGREGATE VALUE DETERMINATION UNIT

96

AGGREGATION DEGREE CALCULATION UNIT

97

AGGREGATION DEGREE OUTPUT UNIT

EP 4 310 497 A1

# FIG. 17

```
                    ┌─────────────┐
                    │    START    │
                    └─────────────┘
                           │
                           ▼                               S1
┌─────────────────────────────────────────────┐
│ OBTAIN RESPECTIVE DETECTION VALUES OF        │
│ SINGLE BEADS AND CLOSE BEADS UP TO           │
│ n-BEADS, FROM PULSE DETECTION CIRCUIT        │
└─────────────────────────────────────────────┘
           │                                │
           ▼  S2                            ▼  S5
┌──────────────────────────┐   ┌──────────────────────────┐
│ IN ONE REACTION REGION,  │   │ SET DENSITY λ OF BEADS   │
│ CALCULATE MEASURED       │   │ IN UNIT SECTIONS TO      │
│ AGGREGATE VALUES C1 TO Cn│   │ PREDETERMINED VALUE      │
│ OF UNIT SECTIONS OF      │   └──────────────────────────┘
│ SINGLE BEADS TO n-BEADS  │                │  S6
└──────────────────────────┘   ┌──────────────────────────┐
           │  S3               │ CALCULATE THEORETICAL    │
           ▼                   │ AGGREGATE VALUES P0 TO Pn│
┌──────────────────────────┐   │ OF UNIT SECTIONS IN      │
│ CALCULATE MEASURED       │   │ WHICH NO BEADS EXIST AND │
│ AGGREGATE VALUE C0 OF    │   │ UNIT SECTIONS IN WHICH   │
│ UNIT SECTIONS IN         │   │ SINGLE BEADS TO n-BEADS  │
│ WHICH NO BEADS EXIST     │   │ EXIST, BASED ON EQUATION │
└──────────────────────────┘   │ ACCORDING TO POISSON     │
           │  S4               │ DISTRIBUTION             │
           ▼                   └──────────────────────────┘
┌──────────────────────────┐                │  S7
│ CREATE ARRAY DATA OF     │   ┌──────────────────────────┐
│ MEASURED AGGREGATE       │   │ CREATE ARRAY DATA OF     │
│ VALUES C0 TO Cn          │   │ THEORETICAL AGGREGATE    │
└──────────────────────────┘   │ VALUES P0 TO Pn          │
                               └──────────────────────────┘
```

S8

CALCULATE ERROR BETWEEN ARRAY DATA OF MEASURED AGGREGATE VALUES C0 TO Cn AND ARRAY DATA OF THEORETICAL AGGREGATE VALUES P0 TO Pn

S9

HAS DENSITY λ AT WHICH ERROR IS MINIMIZED BEEN OBTAINED?  —NO

YES

S10

DETERMINE DENSITY λ AT WHICH ERROR IS MINIMIZED AS DENSITY λfix

S21

CALCULATE AGGREGATION DEGREE

S22

OUTPUT AGGREGATION DEGREE

```
                    ┌─────────────┐
                    │     END     │
                    └─────────────┘
```

# FIG. 18

| | | |
|---|---|---|
| **INTERNATIONAL SEARCH REPORT** | International application No. | |
| | **PCT/JP2021/046699** | |

**A.    CLASSIFICATION OF SUBJECT MATTER**

*G01N 33/543*(2006.01)i; *G01N 21/41*(2006.01)i; *G01N 21/55*(2014.01)i
FI:    G01N21/41 Z; G01N33/543 541Z; G01N21/55

According to International Patent Classification (IPC) or to both national classification and IPC

**B.    FIELDS SEARCHED**

Minimum documentation searched (classification system followed by classification symbols)

G01N33/48-G01N33/98; G01N21/00-G01N21/74; G01N15/00-15/14

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII)

**C.    DOCUMENTS CONSIDERED TO BE RELEVANT**

| Category* | Citation of document, with indication, where appropriate, of the relevant passages | Relevant to claim No. |
|---|---|---|
| A | JP 2020-20668 A (JVC KENWOOD CORPORATION) 06 February 2020 (2020-02-06) entire text, all drawings | 1-6 |
| A | US 5247461 A (PARTICLE DATA, INC.) 21 September 1993 (1993-09-21) entire text, all drawings | 1-6 |
| A | US 5319575 A (TRC COMPANIES, INC.) 07 June 1994 (1994-06-07) entire text, all drawings | 1-6 |
| A | CN 104515725 A (SHENZHEN MINDRAY BIO-MEDICAL ELECTRONICS CO., LTD.) 15 April 2015 (2015-04-15) entire text, all drawings | 1-6 |
| A | JP 2015-194403 A (JVC KENWOOD CORPORATION) 05 November 2015 (2015-11-05) entire text, all drawings | 1-6 |

☐ Further documents are listed in the continuation of Box C.    ☑ See patent family annex.

| | |
|---|---|
| *    Special categories of cited documents: | "T"    later document published after the international filing date or priority date and not in conflict with the application but cited to understand the principle or theory underlying the invention |
| "A"    document defining the general state of the art which is not considered to be of particular relevance | |
| "E"    earlier application or patent but published on or after the international filing date | "X"    document of particular relevance; the claimed invention cannot be considered novel or cannot be considered to involve an inventive step when the document is taken alone |
| "L"    document which may throw doubts on priority claim(s) or which is cited to establish the publication date of another citation or other special reason (as specified) | "Y"    document of particular relevance; the claimed invention cannot be considered to involve an inventive step when the document is combined with one or more other such documents, such combination being obvious to a person skilled in the art |
| "O"    document referring to an oral disclosure, use, exhibition or other means | |
| "P"    document published prior to the international filing date but later than the priority date claimed | "&"    document member of the same patent family |

| Date of the actual completion of the international search | Date of mailing of the international search report |
|---|---|
| **17 February 2022** | **01 March 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

International application No.

**PCT/JP2021/046699**

| Patent document cited in search report | | | Publication date (day/month/year) | Patent family member(s) | | | Publication date (day/month/year) |
|---|---|---|---|---|---|---|---|
| JP | 2020-20668 | A | 06 February 2020 | US | 2021/0148808 | A1 | |
| | | | | WO | 2020/026587 | A1 | |
| US | 5247461 | A | 21 September 1993 | (Family: none) | | | |
| US | 5319575 | A | 07 June 1994 | CA | 2035703 | A1 | |
| CN | 104515725 | A | 15 April 2015 | (Family: none) | | | |
| JP | 2015-194403 | A | 05 November 2015 | US | 2017/0010260 | A1 | |
| | | | | WO | 2015/151752 | A1 | |
| | | | | EP | 3128311 | A1 | |
| | | | | CN | 106332551 | A | |

Form PCT/ISA/210 (patent family annex) (January 2015)

**EP 4 310 497 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2020020668 A
- JP 2021044920 A
- JP 2021044933 A